# EUROPÄISCHE PATENTANMELDUNG

(11) **EP 1 298 206 A1**
(43) Veröffentlichungstag der Anmeldung: **02.04.2003**
(21) Anmeldenummer: 02020261.0
(22) Anmeldetag: 11.09.2002
(51) Int. Cl.: C12N 9/64

(54) **Verwendung der den Blutgerinnungsfaktor VII aktivierenden Protease zur Prophylaxe und Therapie von vaso-proliferativen Erkrankungen**

(30) Priorität: 28.09.2001 DE 10148037
(71) Anmelder: Aventis Behring GmbH, 35002 Marburg (DE)
(72) Erfinder: Kannemeier, Christian, 35390 Giessen (DE); Roemisch, Juergen, 2331 Voesendorf (AT); Preissner, Klaus, T., 35394 Giessen (DE); Kanse, Sandip, 35440 Linden-Leihgestern (DE)

(57) **Zusammenfassung**

Die Verwendung der den Blutgerinnungsfaktor VII aktivierenden Protease (FSAP) zur Prophylaxe und/oder Therapie von vaso-proliferativen Erkrankungen oder Tumorerkrankungen wird beschrieben. Insbesondere wird auf den Einsatz der Protease bei Retinopathie, Neuropathie, rheumatischer Arthritis, Psoriasis, Endometriose, Bronchitis (besonders chronischer) oder chronischen Entzündungen im Magen-Darmtrakt hingewiesen.

## Beschreibung

Die Erfindung betrifft die Verwendung der den Blutgerinnungsfaktor VII aktivierenden Protease bei vaso-proliferativen Erkrankungen.

Aus der deutschen Patentanmeldung 199 03 693.4 ist eine aus dem Blutplasma isolierte Protease bekannt, die den Blutgerinnungsfaktor VII aktivieren kann. Auch ein Verfahren zu ihrer Gewinnung, zu ihrem Nachweis und zu ihrer Inaktivierung sowie Arzneizubereitungen, die diese Protease enthalten, sind dort bereits beschrieben. Entsprechend ihren Eigenschaften wird diese Protease als Faktor Sieben Aktivierende Protease (=FSAP) bezeichnet. In der deutschen Patentanmeldung 199 03 693.4 wird außerdem berichtet, dass die FSAP eine Plasminogen-Aktivatoren aktivierende und/oder verstärkende Wirkung aufweist, die durch die Aktivierung der Einketten-Urokinase (scu PA, single chain urokinase plasminogen activator) oder der Einketten tPA (sctPA, single chain tissue plasminogen activator) gemessen wird.

In der deutschen Patentanmeldung 199 03 693.4 sind auch bereits Verfahren und Testsysteme zum qualitativen und quantitativen Nachweis von FSAP beschrieben, die auf einer Messung, der die Blutgerinnungszeiten verkürzenden Wirkung und der Plasminogen-Aktivatoren aktivierenden Wirkungen von FSAP beruhen. Derartige Testsysteme erlauben auch die Messung von FSAP in komplexen Proteinlösungen wie Plasma, wie es insbesondere in der deutschen Patentanmeldung 199 26 531.3 beschrieben ist.

Es ist außerdem bereits bekannt, dass FSAP die Hämostase und die damit verbundenen zellulären Prozesse beeinflusst. Durch Involvierung in die Blutgerinnung und/oder die Fibrinolyse wirkt es auch auf die Wundheilungsreaktion. Andere bisher bekannte Charakteristika sind die starke Bindung an Hyaluronsäure und Glycosaminoglycane sowie die schnelle Autoaktivierung des Proenzyms in Gegenwart von zum Beispiel Heparin oder Dextransulfat. Die Eigenschaft, an negativ geladene Oberflächen zu binden, und die hohe Affinität zu Heparin machen eine Interaktion der Protease mit Zelloberflächen und extrazellulärer Matrix wahrscheinlich. Charakteristika der zellulären Bindung und resultierende, funktionelle Effekte sind jedoch bisher nicht bekannt.

In DE 100 52 319.6, deren Inhalt hiermit ausdrücklich in diese Anmeldung mit einbezogen wird, wird eine Mutante der FSAP beschrieben, deren Potenz zur Aktivierung von Faktor VII nicht, oder nicht wesentlich eingeschränkt ist. Solche Mutanten sind im Sinne dieser Anmeldung ebenfalls unter dem Begriff FSAP subsummiert.

Es wurde nun gefunden, dass die Proliferation von Gefäßwandzellen durch Inkubation mit FSAP signifikant reduziert werden kann. Dieser Effekt wurde besonders in Gegenwart der aktivierten Protease beobachtet. Die Inhibition von FSAP durch beispielsweise Aprotinin führte zur Reduzierung dieses Effektes. Entsprechend hat das Proenzym eine nur schwache Wirkung auf die Proliferation von Gefäßwandzellen.

Gegenstand der Erfindung ist deshalb die Verwendung der den Blutgerinnungsfaktor aktivierenden Protease (FSAP) zur Prophylaxe und/oder Therapie von vaso-proliferativen Erkrankungen oder Tumorerkrankungen. Die hierfür eingesetzte Protease FSAP kann aus Blutplasma isoliert oder transgen oder rekombinant hergestellt sein.

Zellproliferation ist ein physiologischer Vorgang bei Wachstumsprozessen normaler und transformierter Zellen, die im Tumorgeschehen unkontrolliert ablaufen. Eine dysregulierte Proliferation oder Teilung von Zellen kann durch die bestehende Eigenschaft der aktivierten Form von FSAP gehemmt werden und als Prophylaxe oder Therapie von zum Beispiel Gefäßerkrankungen oder lymphoproliferativen Krankheiten dienen.

Proliferierende glatte Muskelzellen der Gefäßwände tragen zum Beispiel zur Ausprägung der Arteriosklerose bei, die als Wegbereiter von akut eintretenden thrombotischen und thromboembolischen Komplikationen wie Herzinfarkt oder Lungenembolie gilt. Wie im Beispiel unten erläutert, reduziert FSAP signifikant die Proliferation von humanen glatten Muskelzellen, wenn diese mit einem Wachstumsfaktor wie Platelet-derived-growth-factor (PDGF) stimuliert werden, der auch an der Pathogenese von Gefäßerkrankungen beteiligt ist.

Eine vermehrte Proliferation von Gefäßwandzellen beobachtet man auch nach Organtransplantationen, wobei die immunologische Abstoßung meistens durch immunsupprimierende Substanzen verhindert oder hinausgezögert werden kann. Die Gefäßverengung durch Proliferation von Gefäßwandzellen führt zu einer Unterversorgung des Organs, zu thrombotischen Komplikationen und letztendlich zum Organverlust. Entsprechend könnte FSAP auch zur Verhinderung und Abstoßung nach Transplantationen eingesetzt werden.

Darüber hinaus kann FSAP auch zur Prophylaxe oder Therapie von Tumorerkrankungen in zweifacher Weise Verwendung finden: Zum einen spielt die ungehemmte Proliferation von Tumorzellen bei der Krebsentstehung eine dominante Rolle, auf der anderen Seite trägt die Proliferation von Endothelzellen und die damit verbundene Gefäßneubildung (Angiogenese) zum Wachstum vieler vor allem solider Tumoren bei. Eine Reduktion des Wachstums sowohl der Tumorzellen als auch der Vaskularisierung des Tumors über die antiproliferative Wirkung von FSAP kann zu einer Verminderung der Tumormasse führen, und damit potentiell den Stillstand des Wachstums oder eine Tumor-Regression herbeiführen. In ähnlicher Weise kann FSAP zur Therapie (vaso-)proliferativer Erkrankungen und der damit verbundenen Endothelproliferation bei zum Beispiel Retinopathie, Neuropathie, rheumatischer Arthritis, Psoriasis, Endometriose, Bronchitis (besonders chronischer), oder chronischen Entzündungen des Magen-Darmtraktes eingesetzt werden.

### Beschreibung der Abbildung:

Abb. 1 zeigt die Ergebnisse eines Proliferationstestes in Abhängigkeit von FSAP, sowie Kontrollen.

Abb.2 zeigt die Ergebnisse eines Proliferationstestes in Abhängigkeit von verschiedenen pro-FSAP Konzentrationen.

Die anti-proliferative Wirkung von FSAP wird durch das folgende Beispiel gezeigt:

### Beispiel:

### Proliferationsassay

Die Proliferationsrate von humanen glatten Muskelzellen wurde mittels eines DNA-Synthese Testes (Roche Molecular Biochemicals, Mannheim, Deutschland) durchgeführt. Dazu wurden in einer 96 well Mikrotiterplatte 10.000 menschliche glatte Gefäßmuskelzellen je Vertiefung ausgesät. Zwei Tage wurden die Zellen in Dulbecco's modified eagle medium (DMEM) mit 10% fötalem Kälberserum (FCS), 2 mM Glutamin, 100 Einheiten/ml Penicillin/Streptomycin, 1 mM Natrium-pyruvat, 4 g/l Glucose bei 37°C kultiviert und anschließend in DMEM ohne Inhaltsstoffe für zwei weitere Tage bei 37°C inkubiert. Danach wurden die Zellen in DMEM mit 0,2% FCS, 10 ng/ml "Platelet derived growth factor bb" (PDGFbb) überführt und jeweils in Anwesenheit folgender Reagenzien für weitere 24 h bei 37°C inkubiert: 5% FCS (a), Trasylol®, 100 units/ml (b), 0.8 m NaCI, 5 mM Na-Acetat, pH 4.5 (FSAP-buffer) 1:20 verdünnt (c), FSAP buffer 1:20 verdünnt und 100 Einheiten/ml Trasylol® (d), FSAP (aktiviert) in den Konzentrationen 31.3 µg/ml (e), 15.6 µg/ml (f) und 9.1 µg/ml (g), jeweils mit und ohne 100 Einheiten/ml Trasylol® (h-j). Als Kontrolle wurde eine Probe ohne PDGFbb getestet (k) bzw. das oben beschriebene Medium alleine (I). Weiterhin wurde pro-FSAP mit 25 µg/ml (m) 12.5 µg/ml (n) und 6.3 µg/ml (o) unter den oben beschriebenen Bedingungen getestet.

Nach 16 h Inkubationszeit wurde das DNA-Markierungs-Reagenz 5-Brom-2'deoxyuridin (BrdU) in der vom Hersteller angegebenen Verdünnung den Reaktionsansätzen hinzugefügt, und nach 8 h Inkubationszeit bei 37°C wurde der lösliche Zellüberstand extrahiert und die Fixierung nach Angaben des Herstellers durchgeführt. Abweichend vom Protokoll des Herstellers wurden die Vertiefungen der gesamten Mikrotiterplatte mit 3% BSA nach der Fixierung über Nacht bei 4°C blockiert. Anschließend wurde das mit der DNA interkalierte BrdU mit Hilfe eines mit Peroxidase markierten Antikörpers nach den Angaben des Herstellers nachgewiesen.

### Western Blotting:

DMEM mit 0.2% FCS, 20 ng/ml PDGFbb wurde bei 37°C mit 2.5 PEU/ml FSAP bzw. ohne Protease inkubiert. Zu den Zeitpunkten 1 min, 10 min, 30 min, 100 min wurden aus dem Ansatz 75 µl Proben entnommen und mit 25 µl 4-fach konzentrierten, nicht reduzierenden SDS-Proben Puffer gemischt und für 45 min bei 100°C denaturiert. Anschließend wurden die Proben auf ein 1 mm dickes, 10%-iges Polyacrylamid-Gel aufgetragen und für 1 h 45 min bei 100 Volt in einem SDShaltigen Laufpuffer aufgetrennt. Danach wurden die aufgetrennten Proteine in einer Western Blotting Kammer (Bio-Rad, Deutschland) innerhalb von 2 h bei 100 Volt auf eine PVDF-Membran (Amersham Pharmacia) übertragen und anschließend mit 5% Milchpulver in 150 mM NaCI, 50 mM Tris, 0,1% Tween 20 für 1 h bei Raumtemperatur abgesättigt wurde. Nach Inkubation der Membran mit einem gegen PDGFbb gerichteten Kaninchenantikörper (Verdünnung 1:3.000) für 16 h bei 4°C wurde diese dann 3 x mit TBST gewaschen und mit dem Detektions-Antikörper (Schwein-anti-Kaninchen, 1:3.000) für eine Stunde bei 22°C inkubiert. Nach drei weiteren Waschschritten mit TBST für je 5 min wurde zur Detektion der Antikörperbindung ein ECL-Substrat (Amersham Pharmacia, Schweden) eingesetzt, so dass nach Exposition auf Hyperfilm Röntgen-Filmen (Amersham Pharmacia) für 2 min Proteinbanden sichtbar wurden.

### Ergebnisse:

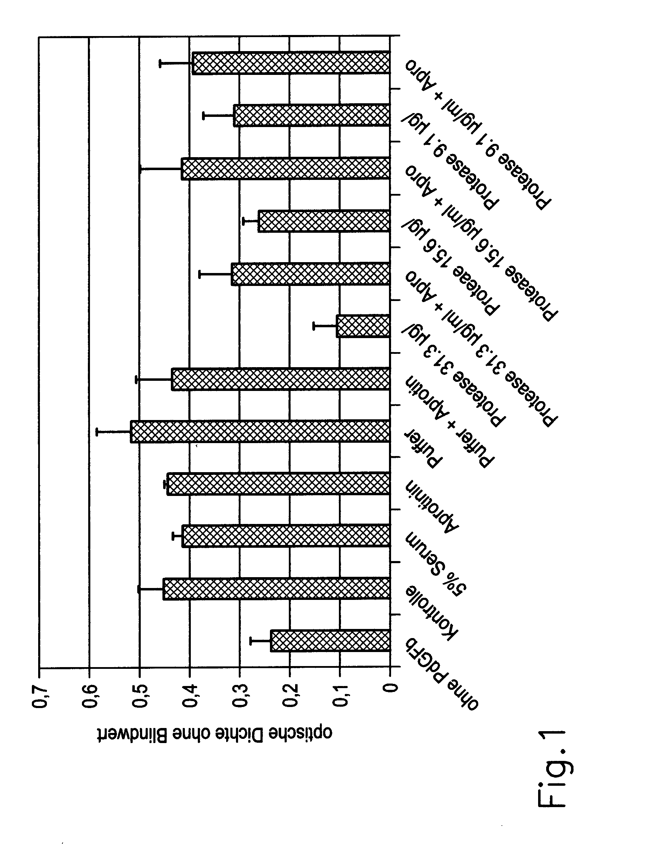

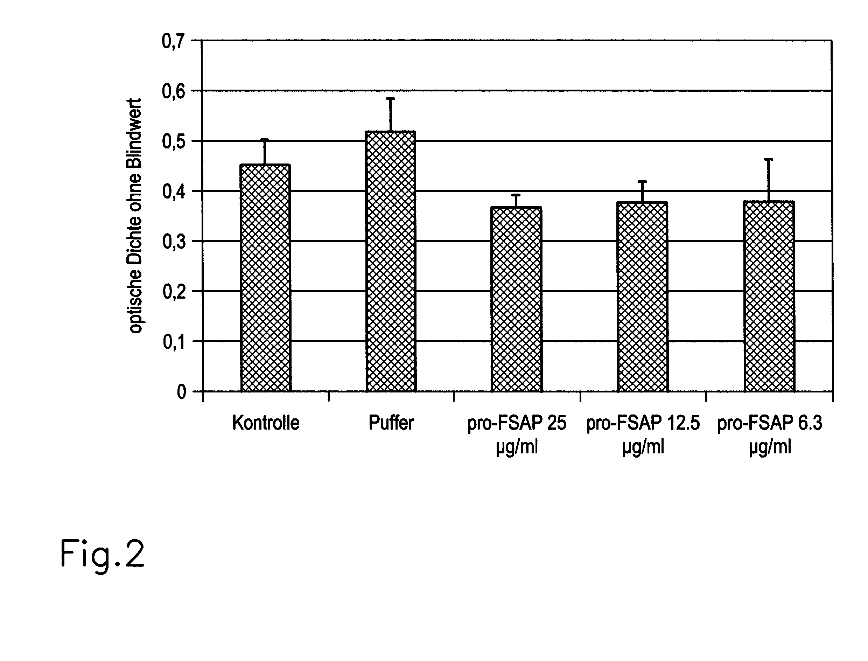

Abb. 1 zeigt exemplarisch das Ergebnis des Proliferationstests. FSAP hemmt in konzentrationsabhängiger Weise die durch PDGFbb stimulierte Proliferation der glatten Muskelzellen, während Puffer alleine keinen Effekt zeigt. Diese antiproliferative Wirkung kann durch Aprotinin fast vollständig aufgehoben werden. Im Gegensatz zu der Protease hat pro-FSAP keine oder nur eine geringe antiproliferative Wirkung (Abb. 2). Nach Koinkubation von PDGFbb mit FSAP ist keine Spaltung von PDGFbb in Western Blot zu erkennen (nicht gezeigt), was eine Inaktivierung des Wachstumsfaktors durch FSAP ausschließt. Entsprechend scheint der FSAP-Effekt auf eine direkte Interaktion der Protease mit den Zellen zurückzuführen zu sein, wobei ein möglicher Rezeptor nicht bekannt ist. Durch diesen neuen Effekt der Protease auf glatte Muskelzellen konnte eine regulatorische oder inhibitorische Funktion der Protease beispielsweise bei unterschiedlichen vasoproliferativen Prozessen nachgewiesen werden.
Gegenstand der Erfindung ist deshalb auch eine pharmazeutische Zubereitung, die eine zur Prophylaxe und/oder Therapie von vaso-proliferativen Erkrankungen oder Tumorerkrankungen ausreichende Menge an der den Blutgerinnungsfaktor VII aktivierenden Protease enthält.

In der DE 101 31 404.3, deren Inhalt hiermit ausdrücklich in diese Anmeldung mit einbezogen wird, werden stabilisierte Flüssigzubereitungen beschrieben, die als pharmazeutische Zubereitung verwendet werden können.

## Patentansprüche

1. Verwendung der den Blutgerinnungsfaktor VII aktivierenden Protease (FSAP) zur Prophylaxe und/oder Therapie von vaso-proliferativen Erkrankungen oder Tumorerkrankungen.

2. Verwendung der den Blutgerinnungsfaktor VII aktivierenden Protease (FSAP) nach Anspruch 1 zur Prophylaxe und Therapie der Retinopathie, Neuropathie, rheumatischen Arthritis, Psoriasis, Endometriose, Bronchitis (besonders chronischer) oder chronischen Entzündungen des Magen-Darmtraktes.

3. Verwendung nach den Ansprüchen 1 und 2, **dadurch gekennzeichnet, dass** eine aus dem Blutplasma isolierte oder transgen oder rekombinant hergestellte, den Blutgerinnungsfaktor VII aktivierende Protease eingesetzt wird.

4. Pharmazeutische Zubereitung, **dadurch gekennzeichnet, dass** sie eine zur Prophylaxe und/oder Therapie der in den Ansprüchen 1 und 2 genannten Erkrankungen ausreichende Menge an der den Blutgerinnungsfaktor VII aktivierenden Protease enthält.
